# EUROPÄISCHE PATENTSCHRIFT

(11) **EP 3 188 778 B1**
(45) Veröffentlichungstag und Bekanntmachung des Hinweises auf die Patenterteilung: **07.11.2018**
(21) Anmeldenummer: 15766419.4
(22) Anmeldetag: 03.09.2015
(51) Int. Cl.: A61M 5/20, A61M 5/24, A61M 5/315

(54) **PEN-TYPE INJECTOR**
PEN-TYPE INJECTOR
INJECTEUR DE TYPE STYLO

(30) Priorität: 05.09.2014 DE 102014217773
(43) Veröffentlichungstag der Anmeldung: 12.07.2017
(73) Patentinhaber: Vetter Pharma-Fertigung GmbH & Co. KG, 88212 Ravensburg (DE)
(72) Erfinder: ROEDLE, Tilman, 88364 Wolfegg (DE); KISTLER, Tobias, 88276 Berg (DE); KÜHNLE, Sarah, 88045 Friedrichshafen (DE); LIMBECK, Roland, 88400 Biberach (DE)
(74) Vertreter: Gleiss, Alf-Olav
(86) Internationale Anmeldenummer: PCT/EP2015/070179
(87) Internationale Veröffentlichungsnummer: WO 2016/034683

(56) Entgegenhaltungen:
- WO-A1-94/13344
- GB-A- 2 471 304
- US-B1- 6 224 567

## Beschreibung

Die Erfindung betrifft einen Pen mit einer Karpule und einem diese aufnehmenden Gehäuse gemäß Oberbegriff des Anspruchs 1.

Pens der hier angesprochenen Art sind bekannt. Sie dienen dazu, einem Patienten ein Medikament zu verabreichen. Sie sind häufig so ausgelegt, dass der Patient die Verabreichung mittels des Pens selbst durchführen kann. Die Karpule derartiger Pens weist einen Hohlraum auf, der von einem verlagerbaren Stopfen verschlossen wird. Durch Verlagerung des Stopfens wird das Medikament aus dem Hohlraum ausgetrieben und einem Patienten verabreicht. Hier angesprochen sind Pens mit einem Antrieb, der dazu dient, den Stopfen innerhalb des Hohlraums vorzugsweise um einen bestimmten Weg zu verlagern, um ein definiertes Volumen eines Medikaments zu verabreichen. Es hat sich herausgestellt, dass die Größe der Pens wesentlich den Komfort der Verwendung derartiger Applikationsmittel beeinträchtigt. Bevorzugt werden daher Pens, die relativ klein ausgelegt sind. Dies hat zum Nachteil, dass dadurch nur eine begrenzte Menge eines Medikaments verfügbar ist.

Aufgabe der Erfindung ist es daher, einen Pen zu schaffen, welcher ein relativ großes Volumen eines Medikaments aufnehmen kann.

Zur Lösung dieser Aufgabe wird ein Pen der oben genannten Art vorgeschlagen, der die Merkmale des Anspruchs 1 aufweist. Er zeichnet sich dadurch aus, dass er so ausgelegt ist, dass die Karpule bei Betätigung des Antriebs zur Verlagerung des Stopfens innerhalb des Innenraums der Karpule in das Gehäuse des Pens eingefahren wird. Dies führt dazu, dass der Pen bei jeder Applikation eines Medikaments - in Richtung seiner Längsausdehnung gesehen - kleiner wird und daher lediglich in seiner Ausgangsposition relativ groß ist. Er ist also zumindest nach der ersten Applikation aber auch, falls die Karpule als Doppelkammerkarpule ausgelegt ist, nach der Rekonstruktion einer in dieser vorhandenen Substanz, kürzer als herkömmliche Pens und kann daher unauffällig von einem Benutzer mitgeführt werden.

Bevorzugt wird ein Ausführungsbeispiel des Pens, welches sich dadurch auszeichnet, dass er eine Hülse aufweist, in welche die Karpule einsetzbar ist. Bei Betätigung des Antriebs des Pens wird die die Karpule aufnehmende Hülse in das Gehäuse des Pens eingezogen, sodass sich seine Länge reduziert. Die Hülse hat den Vorteil, dass die Karpule geschützt ist und nicht so leicht beschädigt werden kann.

Ein bevorzugtes Ausführungsbeispiel des Pens zeichnet sich dadurch aus, dass der Antrieb ein Antriebselement aufweist, welches mit mindestens einem Reibschlusselement versehen ist. Dieses wirkt auf die Karpule oder auf die Hülse so ein, dass diese in das Innere des Gehäuses des Pens verlagert wird, wenn ein Medikament ausgetragen wird. Diese Art der Ausgestaltung ist deshalb besonders günstig, weil die Karpule beziehungsweise die Hülse nicht speziell angepasst sein muss, um mittels eines derartigen Antriebs in das Gehäuse eingefahren zu werden. Das Reibschlusselement kann mit jeder Art von Karpulen und Hülsen zusammenwirken, um die Verlagerung der Karpule zu bewirken.

Bei einem weiteren bevorzugten Ausführungsbeispiel ist vorgesehen, dass der Antrieb ein Antriebselement aufweist, welches mindestens ein erstes Formschlusselement umfasst und dass die Karpule oder die Hülse mit wenigstens einem zweiten Formschlusselement ausgestattet ist. Die beiden Formschlusselemente wirken so miteinander, dass bei Aktivierung des Antriebs die Karpule in das Innere des Gehäuses des Pens verlagert wird. Bei diesem Ausführungsbeispiel ist also die Karpule beziehungsweise die Hülse auf ihrer Außenseite speziell ausgeformt, um ein zweites Formschlusselement zu realisieren, welches mit dem ersten Formschlusselement zusammenwirkt. Die Karpule beziehungsweise die Hülse müssen also auf das erste Formschlusselement abgestimmt sein. Falls die Hülse mit wenigstens einem Formschlusselement ausgestattet ist, kann eine übliche Karpule in diese eingesetzt werden, ohne dass dann die Karpule auf das Antriebs-element mit dem ersten Formschlusselement abgestimmt sein müsste.

Besonders bevorzugt wird ein Ausführungsbeispiel, bei dem das Antriebselement als Gewindehülse mit mindestens einem Innengewindeabschnitt ausgebildet ist. Die Gewindehülse ist innerhalb des Gehäuses durch den Antrieb in Rotation versetzbar und dabei so gelagert, dass sie - in Längsrichtung des Gehäuses gesehen - lagefest gehalten wird. Entsprechend ist die Karpule oder die Hülse auf ihrer Außenseite mit mindestens einem Außengewindeabschnitt versehen, der das zweite Formschlusselement bildet und mit dem Innengewinde des ersten Formschlusselements zusammenwirkt. Die Karpule beziehungsweise die Hülse sind drehfest in dem Gehäuse gehalten, sodass bei Aktivierung des Antriebselements, also der Gewindehülse, die Karpule allein oder mit der Hülse in das Gehäuse des Pens eingezogen wird.

Ganz besonders bevorzugt wird ein Ausführungsbeispiel des Pens, welches sich dadurch auszeichnet, dass mindestens ein Sensor vorgesehen ist, welcher die Relativposition der Karpule gegenüber dem Gehäuse erfasst, insbesondere die Relativposition des mindestens einen Stopfens gegenüber der Kolbenstange. Es ist damit möglich, beim Einsetzen der Karpule, gegebenenfalls gemeinsam mit einer Hülse, eine definierte Ausgangsposition zu erfassen, aufgrund derer sichergestellt werden kann, dass bereits die erste Applikation eines Medikaments mit einem exakt definierten Volumen erfolgt.

Weitere Ausgestaltungen ergeben sich aus den übrigen Unteransprüchen.

Die Erfindung wird im Folgenden anhand der Zeichnung näher erläutert. Es zeigen:
- Figur 1: einen Längsschnitt eines Pens in einer ersten Funktionsstellung und
- Figur 2: einen Längsschnitt durch den Pen gemäß Figur 1 in einer zweiten Funktionsstellung.

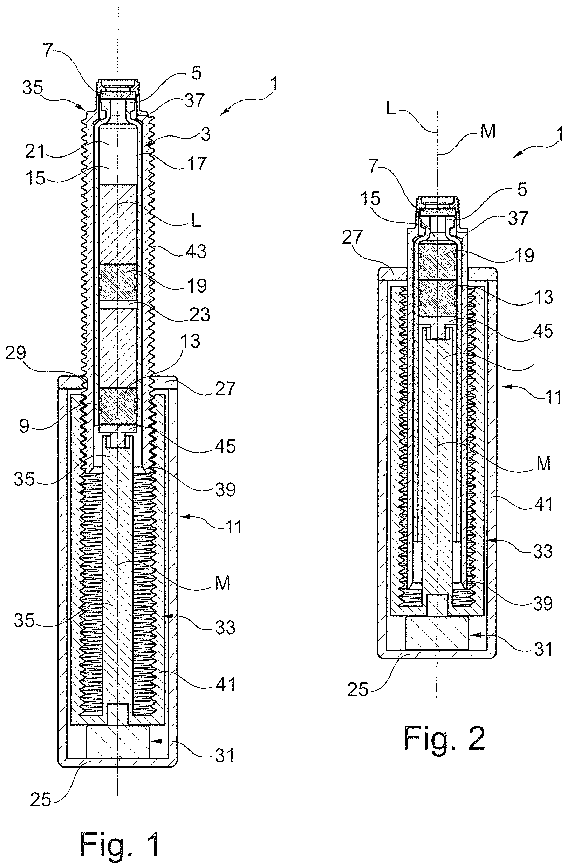

Figur 1 zeigt im Längsschnitt einen Pen 1 mit einer Karpule 3. Diese weist ein erstes Ende 5 auf, welches in Figur 1 oben angeordnet ist und mit einem Verschlussteil 7 verschlossen ist. Das gegenüberliegende, in Figur 1 unten liegende zweite Ende 9 der Karpule 3 liegt im Inneren eines Gehäuses 11 des Pens 1. Die Karpule 3 ist also mit dem zweiten Ende 9 voraus in das Gehäuse 11 einsetzbar. Die Karpule 3 wird hier durch einen in deren Inneres eingeführten ersten Stopfen 13 abgeschlossen, sodass zwischen dem Verschlussteil 7 und dem ersten Stopfen 13 ein Innenraum 15 der Karpule 3 definiert wird. Dieser wird durch das Verschlussteil 7 und den ersten Stopfen 13 dicht abgeschlossen.

Bei einer Karpule 3, wie sie in Zusammenhang mit einem Pen 1 verwendet werden kann, kann es sich um eine bekannte Ein-Kammer-Karpule handeln, die einen zylindrischen Körper aufweist, der zwischen dem Verschlussteil 7 und dem hier dargestellten ersten Stopfen 13 durchgehend ausgebildet ist und ein zu verabreichendes Medikament enthält.

Hier dargestellt ist eine bekannte Doppelkammerkarpule, die üblicherweise einen zylindrischen Grundkörper 17 umfasst, wobei dieser den Innenraum 15 einschließt. Bei Doppelkammerkarpulen ist außer dem ersten Stopfen 13 am unteren, zweiten Ende 9 ein zweiter Stopfen 19 vorgesehen, der den Innenraum 15 in einen oberen Teilraum 21 und einen unteren Teilraum 23 unterteilt. In dem unteren Teilraum 23 ist üblicherweise eine erste Komponente eines Medikaments, beispielsweise ein Lösungsmittel, untergebracht, während sich in dem oberen Teilraum 21 eine zweite Komponente des Medikaments befindet, beispielsweise ein Lyophilisat. Es können auch beide Teilräume 21, 23 eine Flüssigkeit enthalten, die vor Verwendung des Pens 1 gemischt werden. Zur Aktivierung beziehungsweise Restituierung des Lyophilisats wird der erste Stopfen 13 innerhalb des Innenraums 15 nach oben verlagert. Dadurch wird im unteren Teilraum 23 ein Überdruck aufgebaut, der dazu führt, dass der zweite Stopfen 19 ebenfalls nach oben verschoben wird. Dadurch gelangt der zweite Stopfen 19 in den Bereich eines hier nicht dargestellten Bypasses, sodass das Lösungsmittel an dem zweiten Kolben 19 vorbeiströmen kann und somit beispielsweise aus dem unteren Teilraum 23 in den oberen Teilraum 21 gelangt und das Lyophilisat auflöst. Die Karpule kann zur Verbesserung des Lösungsvorgangs geschüttelt werden. Der Aufbau und die Funktionsweise beziehungsweise Aktivierung einer Doppelkammerkarpule sind bekannt, sodass hier nicht näher darauf eingegangen wird.

Der hier dargestellte Pen 1 weist also nach allem eine Karpule 13 auf, sei es eine Ein-Kammer- oder eine Doppelkammerkarpule, wie in Figur 1 dargestellt, und mindestens einen Kolben, hier den ersten Stopfen 13 auf. Doppelkammerkarpulen sind zusätzlich mit dem hier dargestellten zweiten Stopfen 19 versehen.

Das in Figur 1 dargestellte Ausführungsbeispiel des Pens 1 weist ein Gehäuse 11 auf, welches im Wesentlichen zylindrisch ausgebildet ist und an seinem hier unteren Ende einen Boden 25 und an seinem in Figur 1 dargestellten oberen Ende einen Deckel 27 mit einer Öffnung 29 umfasst, durch welche die Karpule 3 in das Innere des Gehäuses 11 einführbar ist.

Innerhalb des Gehäuses 11, vorzugsweise im Bereich des Bodens 25, befindet sich ein Antrieb 31, der eine hier nicht dargestellte Energieversorgung aufweist.

Der Antrieb 31 dient dazu, die Karpule 3 in das Innere des Gehäuses 11 zu verlagern, wenn ein im Innenraum 15 vorhandenes Medikament verabreicht werden soll. Um die Medikamentenabgabe zu ermöglichen, muss das Verschlussteil 7 von der Karpule 3 mit einer üblichen Kanüle durchstochen werden.

Entscheidend ist, dass bei der Verlagerung der Karpule 3 in das Innere des Gehäuses 11 der erste Stopfen 13 bezogen auf die Längsachse L der Karpule 3 und die koaxial dazu angeordnete Mittelachse M in den Innenraum 15 verlagert wird. Um eine Verlagerung der Karpule 3 in das Innere des Gehäuses 11 zu bewirken, um also die Karpule 3 aus der in Figur 1 dargestellten oberen ersten Funktionsstellung nach unten zu verlagern, kann der Antrieb 31 ein in Figur 1 nicht dargestelltes Antriebselement aufweisen, welches mit der Karpule 3 über Reib- oder Formschluss so zusammenwirkt, dass diese bei Aktivierung des Antriebs in Richtung ihrer Längsachse L und damit in Richtung der Mittelachse M nach unten in das Gehäuse 11 hinein verlagert wird.

Der hier dargestellte Pen 1 weist eine im Inneren des Gehäuses 11 koaxial zur Mittelachse M und zur Längsachse L angeordnete Kolbenstange 34 auf, die, in Richtung der Mittelachse M gesehen, lagefest im Gehäuse 11 angeordnet ist. Sie kann also während die Karpule 3 in das Innere des Gehäuses 11 verlagert wird, nicht nach unten ausweichen. Dadurch findet eine Relativbewegung des oberen Endes 35 der Kolbenstange 34 gegenüber der Karpule 3 statt, während diese in das Gehäuse 11 eingezogen wird. Das erste Ende 5 der Karpule 3 bewegt sich somit in Richtung auf das obere Ende 35 der Kolbenstange 34, sodass auch der erste Stopfen 13 in dem Innenraum 15 nach oben in Richtung auf das erste Ende 5 der Karpule 3 verlagert wird.

Bei dem in Figur 1 dargestellten Ausführungsbeispiel des Pens 1 ist die Karpule 3 innerhalb einer Hülse 36 angeordnet, die oben eine erste Öffnung 37 für das erste Ende 5 der Karpule 3 aufweist, sodass dieses erste Ende 5 zugänglich ist. Die Hülse 36 weist außerdem eine zweite Öffnung 39 an ihrem unteren Ende auf, über welche der mindestens eine Stopfen der Karpule 3, hier also der erste Stopfen 13, zugänglich ist. Für die Grundfunktion des Pens 1, die darauf ausgelegt ist, die Karpule 3 in das Innere des Gehäuses 11 mittels des Antriebs 31 sowie dessen Antriebselements 33 in das Innere des Gehäuses 11 zu verlagern, spielt es keine Rolle, ob das Antriebselement 33 unmittelbar auf die Karpule 3 oder auf die die Karpule 3 aufnehmende Hülse 36 einwirkt.

Für die bei dem hier vorliegenden Pen 1 realisierte Grundidee ist es außerdem nicht von Bedeutung, ob der Antrieb 31 über ein Antriebselement 33 auf die Karpule 3 beziehungsweise die Hülse 36 einwirkt, und dazu mindestens ein Reibschlusselement aufweist, oder ob das Antriebselement 33 mindestens ein Formschlusselement umfasst, welches auf wenigstens ein zweites Formschlusselement an der Karpule 3 oder der Hülse 36 einwirkt, um bei Aktivierung des Antriebs 31 die Karpule 3 in das Innere des Gehäuses 11 zu verlagern, gegebenenfalls gemeinsam mit der Hülse 36. Falls die Karpule 3 im Inneren der Hülse 36 angeordnet ist, ist es nicht erforderlich, die Karpule 3 auf das Antriebselement 33 abzustimmen, weil dieses auf die Hülse 36 einwirkt und diese gemeinsam mit der Karpule 3 in das Innere des Gehäuses 11 verlagert.

Bei dem in Figur 1 dargestellten Ausführungsbeispiel des Pens 1 ist vorgesehen, dass das Antriebselement 33 eine Gewindehülse 41 mit einem Innengewinde aufweist, welches ein erstes Formschlusselement darstellt. Dieses ist hier vorzugsweise durchgehend ausgebildet. Es ist aber sehr wohl ausreichend, auf der Innenseite der Gewindehülse 41 mindestens einen sich in Längsrichtung erstreckenden Gewindeabschnitt vorzusehen. Die Gewindehülse 41 ist mittels des Antriebs 31 in Rotation versetzbar. Sie ist dazu im Inneren des Gehäuses 11 drehbar gelagert, dabei aber so in dem Gehäuse 11 untergebracht, dass sie in Richtung der Mittelachse gesehen, lagefest gehalten wird.

Figur 1 zeigt einen Pen 1, bei dem die Karpule 3 von einer Hülse 36 umgeben ist. Auf deren Außenseite ist ein zweites Formschlusselement vorgesehen, welches als Außengewinde 43 ausgebildet ist.

Bei Aktivierung des Antriebs 31 wird das als Gewindehülse 41 ausgebildete Antriebselement 33 in Rotation versetzt, sodass es sich um die Mittelachse M dreht, bezüglich dieser Achse aber lagefest im Gehäuse 11 gehalten wird. Das das erste Formschlusselement des Antriebselements 33 bildende Innengewinde wirkt mit dem das zweite Formschlusselement bildende Außengewinde 43 auf der Außenseite der Hülse 36 so zusammen, dass die Hülse 36, damit auch die Karpule 3, entlang der Längsachse L beziehungsweise der Mittelachse M in das Innere des Gehäuses 11 verlagert wird. Um die Verlagerung der Karpule 3 in ihrer Längsrichtung bei einer Rotation des Antriebselements 33 zu ermöglichen, ist die Hülse 36 drehfest in dem Gehäuse 11 angeordnet, wobei diese drehfeste Lagerung eine axiale Verlagerung der Karpule 3 in Richtung ihrer Längsachse L zulässt.

Aus den Erläuterungen wird deutlich, dass das zweite Formschlusselement, also ein Außengewinde, auch unmittelbar auf der Außenfläche der Karpule 3 realisiert werden kann. Es ist also zur Realisierung des hier beschriebenen Funktionsprinzips des Pens 1 nicht erforderlich, die Karpule 3 gemeinsam mit einer diese aufnehmenden Hülse 36 zu verwenden. Es hat sich allerdings gezeigt, dass eine Hülse 36 der hier angesprochenen Art sehr wohl geeignet ist, die Karpule 3 des Pens 1 zu schützen. Überdies ist es nicht erforderlich, bei Verwendung einer derartigen Hülse 36 eine Karpule 3 speziell auf den Einsatz in Zusammenhang mit einem Pen 1 der hier angesprochenen Art auszugestalten. Es können also übliche Karpulen in eine hier angesprochene Hülse 36 eingesetzt werden, um den Pen 1 zu realisieren.

Besonders bevorzugt sind das Antriebselement 33 und die Kolbenstange 34 einstückig ausgebildet. Wird also das Antriebselement 33 mithilfe des Antriebs 31 in Rotation versetzt, so dreht sich auch die Kolbenstange 34 synchron mit dem Antriebselement 33 um die Mittelachse M. Zwischen dem oberen Ende der Kolbenstange 34 und dem ersten Stopfen 13 wirken dadurch Reibkräfte, die Nachteile mit sich ziehen können. Um dies zu vermeiden, ist vorzugsweise zwischen dem oberen Ende der Kolbenstange 34 und dem ersten Stopfen 13 eine Drehentkopplung 45 vorgesehen. Diese bewirkt, dass sich eine Drehung der Kolbenstange 34 nicht auf den ersten Stopfen 13 überträgt.

Figur 2 zeigt den Pen 1 in einer zweiten Funktionsstellung, in der die Karpule 3, die hier gemeinsam mit der Hülse 36 verwendet wird, gänzlich in das Innere des Gehäuses 11 eingezogen ist. Gleiche und funktionsgleiche Elemente sind mit gleichen Bezugszeichen versehen, sodass insofern auf die Beschreibung zu Figur 1 verwiesen wird.

In dieser zweiten Funktionsstellung ist der erste Stopfen 13 maximal weit in Richtung auf das erste Ende 5 der Karpule 3 verlagert worden, hier auch der zweite Stopfen 19, sodass ein im Innenraum 15 vorhandenes Medikament vollständig ausgetragen ist.

Bei Einsatz des hier beschriebenen Pens 1 wird bei jeder Applikation des im Innenraum 15 vorhandenen Medikaments die Karpule 3, hier gemeinsam mit der Hülse 36, in das Innere des Gehäuses 11 verlagert, sodass sich die Länge des Pens 1 mit jeder Applikation reduziert. Nur im frisch gefüllten Zustand ist der Pen 1 genauso lang wie herkömmliche Pens. Seine Größe reduziert sich bei der Restitution eines Medikaments und während der Verabreichung eines in der Karpule 3 vorhandenen Medikaments mit jeder Applikation, sodass der Pen 1 leicht verstaut und ohne weiteres mitgeführt werden kann. Die kleineren Abmessungen des Pens 1, in Richtung der Mittelachse M beziehungsweise Längsachse L gesehen, ist also für einen Benutzer äußerst vorteilhaft.

Bei dem anhand der Figuren 1 und 2 beschriebenen Pen ist vorzugsweise ein hier nicht dargestellter Sensor vorgesehen, welcher die Relativposition der Karpule 3 gegenüber dem Gehäuse 11 erfasst, insbesondere die Relativposition des mindestens einen Stopfens 13 gegenüber der Kolbenstange 34. Es ist damit möglich sicherzustellen, dass vor dem ersten Einsatz des Pens 1 die Kolbenstange 34 gegebenenfalls über die vorzugsweise vorgesehene Drehentkopplung 45 an dem ersten Stopfen 13 anliegt. Bei der ersten Betätigung des Pens 1, bei welcher der Antrieb 31 aktiviert und die Karpule 3 in das Gehäuse 11 eingefahren wird, ist eine definierte Ausgangsposition des Endes der Kolbenstange 34 gegenüber dem ersten Stopfen 13 im Inneren der Karpule 3 gegeben, bevor die Karpule 3 verlagert wird. Es wird also eine exakt definierte Menge eines Medikaments aus dem Innenraum 15 der Karpule 3 ausgetragen.

Der Antrieb 31 weist insbesondere einen Elektromotor auf. Dieser ist vorzugsweise mit einem Sensor versehen, der dazu dient, die Einstellung einer definierten Relativposition zwischen der Kolbenstange 34 und dem ersten Stopfen 13 zu ermöglichen und beispielsweise den von dem Elektromotor aufgenommenen Strom erfasst. Sobald dieser ansteigt, weil die Kolbenstange 34 gegebenenfalls über die Drehentkopplung 45 an dem ersten Stopfen 13 anliegt, kann der Antrieb 31 inaktiviert werden. Damit ist sichergestellt, dass sich eine definierte Relativposition ergibt. Damit ist auch gewährleistet, dass bei der ersten Betätigung des Pens 1 ein exakt definiertes Volumen eines Medikaments ausgetragen wird.

Der hier erwähnte Sensor kann also ein den vom Antrieb 31 aufgenommenen Strom erfassender Sensor oder beispielsweise auch ein Asynchronzähler sein, oder aber ein Sensor, der die Position des oberen Endes der Kolbenstange 34 beziehungsweise der Drehentkopplung 45 gegenüber dem ersten Stopfen 13 auf beliebige, bekannte Weise erfasst, sei es auf mechanische oder optische Weise. Auf jeden Fall ist mittels eines hier beschriebenen Sensors eine exakte Ausgangsposition des oberen Endes gegenüber dem mindestens einen Stopfen definierbar, wodurch bereits das erste von dem Pen 1 ausgetragene Volumen eines Medikaments exakt vorherbestimmbar ist. Dies gewährleistet eine sichere Verwendung dieses Pens 1.

## Patentansprüche

1. Pen (1) mit
- einer eine Längsachse (L) aufweisenden Karpule (3), welche
• ein erstes Ende (5) mit einer mittels eines Verschlussteils (7) verschließbaren Abgabeöffnung,
• ein zweites Ende (9), über welches mindestens ein in Richtung der Längsachse (L) verschieblicher Stopfen (13) einführbar ist, und
• einen von dem Verschlussteil (7) und dem mindestens einen Stopfen (13) dicht abgeschlossenen Innenraum (15) aufweist,
- einem eine Mittelachse (M) aufweisenden, die Karpule (3) aufnehmenden Gehäuse (11), welches
• eine mit dem mindestens einen Stopfen (13) zusammenwirkende Kolbenstange (34), und
• einen Antrieb (31) zur Verlagerung des mindestens einen Stopfens (13) innerhalb des Innenraums (15) der Karpule (3) aufweist, wobei
• die Karpule (3) mit dem zweiten Ende (9) voraus in das Gehäuse (11) einsetzbar ist,
**dadurch gekennzeichnet, dass**
- die Kolbenstange (34) innerhalb des Gehäuses (11) - in Richtung der Mittelachse (M) des Gehäuses (11) gesehen - lagefest angeordnet ist,
- der Antrieb (31) ein mit der Karpule (3) zusammenwirkendes Antriebselement (33) aufweist,
- das Antriebselement (33) bei Aktivierung des Antriebs (31) die Karpule (3) in das Gehäuse (11) - in Richtung Ihrer Längsachse (L), die koaxial zur Mittelachse (M) des Gehäuses (11) angeordnet - verlagert, und dass dabei die Kolbenstange (34) in Bezug auf die Längsrichtung des Gehäuses (11) in einer lagefesten Position gehalten wird, sodass
- die in das zweite Ende (9) der Karpule (3) eingreifende Kolbenstange (34) den mindestens einen Stopfen (13) innerhalb des Innenraums (15) der Karpule (3) in Richtung auf deren erstes Ende (5) verlagert.

2. Pen nach Anspruch 1, **dadurch gekennzeichnet, dass** die Karpule (3) als Doppelkammerkarpule ausgelegt ist.

3. Pen nach Anspruch 1 oder 2, **dadurch gekennzeichnet, dass** eine die Karpule (3) aufnehmende Hülse (36) vorgesehen ist, welche eine erste Öffnung (37) für das erste Ende (5) der Karpule (3) und eine zweite Öffnung (39) aufweist, über welche der mindestens eine Stopfen (13) über das zweite Ende (9) der Karpule (3) zugänglich ist, und dass das Antriebselement (33) mit der Hülse (36) so zusammenwirkt, dass bei Aktivierung des Antriebs (31) die Karpule (3) in das Gehäuse (11) hinein verlagerbar ist.

4. Pen nach einem der vorhergehenden Ansprüche, **dadurch gekennzeichnet, dass** das Antriebselement (33) mindestens ein Reibschlusselement aufweist, welches auf die Karpule (3) oder auf die Hülse (36) einwirkt, um die Verlagerung der Karpule (3) herbeizuführen.

5. Pen nach einem der vorhergehenden Ansprüche 1 bis 3, **dadurch gekennzeichnet, dass** das Antriebselement (33) mindestens ein erstes Formschlusselement und die Karpule (3) oder Hülse (36) wenigstens ein zweites Formschlusselement aufweisen, und dass das erste und zweite Formschlusselement so zusammenwirken, dass bei Aktivierung des Antriebs (31) die Karpule (3) in das Gehäuse (11) verlagert wird.

6. Pen nach Anspruch 5, **dadurch gekennzeichnet, dass** das Antriebselement (33) als Gewindehülse (41) mit einem Innengewinde ausgebildet ist, die im Gehäuse (11) rotierbar gelagert und von dem Antrieb (31) in Rotation versetzbar ist, wobei die Gewindehülse (41) innerhalb des Gehäuses (11) derart gelagert ist, dass sie - in Richtung der Mittelachse (M) des Gehäuses (11) gesehen - lagefest gehalten wird, dass die Karpule (3) oder die Hülse (36) auf ihre Außenseite ein mit der Gewindehülse (41) zusammenwirkendes Außengewinde aufweist, und dass die Karpule (3) oder die Hülse (36) drehfest in dem Gehäuse (11) anordenbar ist.

7. Pen nach Anspruch 1, **dadurch gekennzeichnet, dass** zwischen der Kolbenstange (34) und dem mindestens einen Kolben (13) eine Drehentkopplung (45) vorgesehen ist.

8. Pen nach Anspruch 1, **dadurch gekennzeichnet, dass** der Antrieb (31) einen Elektromotor aufweist.

9. Pen nach Anspruch 1, **dadurch gekennzeichnet, dass** er einen Sensor aufweist, welcher die Relativposition der Karpule (3) gegenüber dem Gehäuse (11) erfasst, insbesondere die Relativposition des mindestens einen Stopfens (13) gegenüber der Kolbenstange (34).

10. Pen nach Anspruch 8, **dadurch gekennzeichnet, dass** der Antrieb (31) einen Sensor aufweist, der die Relativposition der mindestens einen Kolbenstange (34) gegenüber dem mindestens einen Stopfen (13) erfasst.

## Claims

1. A pen (1) comprising
- a carpule (3) that has a longitudinal axis (L),
• a first end (5) with a dispensing opening that can be closed by means of a closure part (7),
• a second end (9) via which at least one stopper (13) can be inserted, the stopper (13) being moveable in the direction of the longitudinal axis (L), and
• an interior (15) that is tightly closed by the closure part (7) and the at least one stopper (13),
- a housing (11) that has a central axis (M), receives the carpule (3), and
• has a piston rod (34) that interacts with the at least one stopper (13), and
• has a drive (31) for moving the at least one stopper (13) within the interior (15) of the carpule (3), wherein
• the carpule (3) can be inserted into the housing (11) with the second end (9) leading,
**characterized in that**
- the piston rod (34) is arranged within the housing (11) in a fixed position when seen in the direction of the central axis (M) of the housing (11),
- the drive (31) has a drive element (33) that interacts with the carpule (3),
- the drive element (33) moves the carpule (3) into the housing (11) in the direction of its longitudinal axis (L), which is arranged coaxially to the central axis (M) of the housing (11), when the drive (31) is activated, and in the process the piston rod (34) is held in a fixed position with respect to the longitudinal direction of the housing (11) such that
- the piston rod (34), which is engaged in the second end (9) of the carpule (3), moves the at least one stopper (13) within the interior (15) of the carpule (3) in the direction of the first end (5) of the carpule (3).

2. The pen according to claim 1, **characterized in that** the carpule (3) is designed as a dual-chamber carpule.

3. The pen according to claim 1 or 2, **characterized in that** a sleeve (36) accommodating the carpule (3) is provided that has a first opening (37) for the first end (5) of the carpule (3) and a second opening (39) via which the at least one stopper (13) is accessible via the second end (9) of the carpule (3), and **in that** the drive element (33) interacts with the sleeve (36) such that the carpule (3) may be moved into the housing (11) when the drive (31) is activated.

4. The pen according to any of the preceding claims, **characterized in that** the drive element (33) has at least one friction fit element that acts on the carpule (3) or on the sleeve (36) in order to cause the carpule (3) to move.

5. The pen according to any of the preceding claims 1 through 3, **characterized in that** the drive element (33) has at least a first positive fit element and the carpule (3) or sleeve (36) has at least a second positive fit element, and **in that** the first positive fit element and the second positive fit element interact such that when the drive (31) is activated the carpule (3) is moved into the housing (11).

6. The pen according to claim 5, **characterized in that** the drive element (33) is embodied as a threaded sleeve (41) with an internal thread that is rotatably mounted in the housing (11) and that can be caused to rotate by the drive (31), wherein the threaded sleeve (41) is mounted within the housing (11) such that it is held in a fixed position, seen in the direction of the central axis (M) of the housing (11), that the exterior of the sleeve (36) or the carpule (3) has an external thread that interacts with the threaded sleeve (41), and that the carpule (3) or the sleeve (36) may be arranged torque-proof in the housing (11).

7. The pen according to claim 1, **characterized in that** a rotation decoupling means (45) is provided between the piston rod (34) and the at least one piston (13).

8. The pen according to claim 1, **characterized in that** the drive (31) has an electric motor.

9. The pen according to claim 1, **characterized in that** it has a sensor that detects the relative position of the carpule (3) relative to the housing (11), in particular the relative position of the at least one stopper (13) relative to the piston rod (34).

10. The pen according to claim 8, **characterized in that** the drive (31) has a sensor that detects the relative position of the at least one piston rod (34) relative to the at least one stopper (13).

## Revendications

1. Stylo (1) avec
- une cartouche (3) présentant un axe longitudinal (L), présentant
• une première extrémité (5) avec un orifice de sortie verrouillable au moyen d'un élément de fermeture (7),
• une deuxième extrémité (9) par laquelle au moins un bouchon (13) déplaçable en direction de l'axe longitudinal (L) peut être introduit et
• un espace intérieur (15) fermé hermétiquement par l'élément de fermeture (7) et l'au moins un bouchon (13),
- un boîtier (11) récepteur de la cartouche (3), présentant un axe central (M), qui présente
• une tige de piston (34) interagissant avec l'au moins un bouchon (13) et
• un entraînement (31) pour le déplacement de l'au moins un bouchon (13) à l'intérieur de l'espace intérieur (15) de la cartouche (3),
• la cartouche (3) étant utilisable préalablement avec la deuxième extrémité (9) dans le boîtier (11),
**caractérisé en ce que**
- la tige de piston (34) est disposée dans une position fixe à l'intérieur du boîtier (11) - vu en direction de l'axe central (M) du boîtier (11),
- l'entraînement (31) présente un élément d'entraînement (33) interagissant avec la cartouche (3),
- l'élément d'entraînement (33) déplace la cartouche (3) dans le boîtier (11) lorsque l'entraînement (31) est activé - en direction de son axe longitudinal (L) qui est disposé coaxialement par rapport à l'axe central (M) du boîtier (11) - et que la tige de piston (34) est maintenue dans une position fixe quant au sens longitudinal du boîtier (11) de telle sorte que
- la tige de piston (34) s'engageant dans la deuxième extrémité (9) de la cartouche (3) déplace l'au moins un bouchon (13) à l'intérieur de l'espace intérieur (15) de la cartouche (3) en direction de sa première extrémité (5).

2. Stylo selon la revendication 1, **caractérisé en ce que** la cartouche (3) est conçue comme cartouche à double compartiment.

3. Stylo selon la revendication 1 ou 2, **caractérisé en ce qu'**une douille (36) récepteur de la cartouche (3) qui présente un premier orifice (37) pour la première extrémité (5) de la cartouche (3) et un deuxième orifice (39), par lequel l'au moins un bouchon (13) est accessible par la deuxième extrémité (9) de la cartouche (3), est prévu et que l'élément d'entraînement (33) interagit avec la douille (36) de manière à ce que la cartouche (3) soit déplaçable dans le boîtier (11) lorsque l'entraînement (31) est activé.

4. Stylo selon l'une des revendications précédentes, **caractérisé en ce que** l'élément d'entraînement (33) présente au moins un élément d'engagement à friction qui agit sur la cartouche (3) ou sur la douille (36) pour induire le déplacement de la cartouche (3).

5. Stylo selon l'une des revendications précédentes 1 à 3, **caractérisé en ce que** l'élément d'entraînement (33) présente au moins un premier élément à ajustement de forme et la cartouche (3) ou la douille (36) présente au moins un deuxième élément à ajustement de forme et que les premier et deuxième éléments à ajustement de forme interagissent de manière à ce que la cartouche (3) soit déplacée dans le boîtier (11) lorsque l'entraînement (31) est activé.

6. Stylo selon la revendication 5, **caractérisé en ce que** l'élément d'entraînement (33) est conçu comme douille filetée (41) avec un filetage intérieur reposant rotativement dans le boîtier (11) et déplaçable en rotation par l'entraînement (31), la douille filetée (41) reposant à l'intérieur du boîtier (11) de manière à être maintenu dans une position fixe - vu en direction de l'axe central (M) du boîtier (11), que la cartouche (3) ou la douille (36) présente, sur sa face extérieure, un filetage extérieur interagissant avec la douille filetée (41) et que la cartouche (3) ou la douille (36) peut être disposée de façon solidaire en rotation dans le boîtier (11).

7. Stylo selon la revendication 1, **caractérisé en ce qu'**un découplage rotatif (45) est prévu entre la tige de piston (34) et l'au moins un piston (13).

8. Stylo selon la revendication 1, **caractérisé en ce que** l'entraînement (31) présente un moteur électrique.

9. Stylo selon la revendication 1, **caractérisé en ce qu'**il présente un capteur qui enregistre la position relative de la cartouche (3) par rapport au boîtier (11), notamment la position relative de l'au moins un bouchon (13) par rapport à la tige de piston (34).

10. Stylo selon la revendication 8, **caractérisé en ce que** l'entraînement (31) présente un capteur qui enregistre la position relative de l'au moins une tige de piston (34) par rapport à l'au moins un bouchon (13).
